# EUROPEAN PATENT SPECIFICATION

(11) **EP 2 397 541 B1**
(45) Date of publication and mention of the grant of the patent: **22.07.2015**
(21) Application number: 10166236.9
(22) Date of filing: 17.06.2010
(51) Int. Cl.: C12N 1/12

(54) **A method for harvesting algae**
Verfahren zum Ernten von Algen
Procédé pour la collecte d'algues

(43) Date of publication of application: 21.12.2011
(73) Proprietor: Neste Oil Oyj, 02150 Espoo (FI)
(72) Inventor: Malm, Annika, 00510, Helsinki (FI); Tanner, Reijo, 12240, Hikiä (FI)
(74) Representative: Zacco Denmark A/S

(56) References cited:
- US-A1- 2009 162 919
- MOLINA GRIMA E ET AL: "Recovery of microalgal biomass and metabolites: process options and economics" BIOTECHNOLOGY ADVANCES, ELSEVIER PUBLISHING, BARKING, GB LNKD- DOI:10.1016/S0734-9750(02)00050-2, vol. 20, no. 7-8, 1 January 2003 (2003-01-01), pages 491-515, XP004400158 ISSN: 0734-9750
- ANDREW K LEE ET AL: "Microbial flocculation, a potentially low-cost harvesting technique for marine microalgae for the production of biodiesel" JOURNAL OF APPLIED PHYCOLOGY, KLUWER ACADEMIC PUBLISHERS, DO, vol. 21, no. 5, 13 December 2008 (2008-12-13), pages 559-567, XP019749634 ISSN: 1573-5176
- LUBIAN L M: "CONCENTRATING CULTURED MARINE MICROALGAE WITH CHITOSAN" AQUACULTURAL ENGINEERING, vol. 8, no. 4, 1989, pages 257-266, XP002594680 ISSN: 0144-8609

## Description

### Field of the invention

The present invention relates to a method for harvesting algae from an aqueous solution. In particular, the present invention relates to a robust method capable of effectively harvesting algae even from dilute aqueous solutions with good yield.

### Background of the invention

Algae are an attractive alternative for producing renewable oil due to its common nature and high production potential. Especially, as algae may be grown in various kinds of aqueous systems, even in sewage waters, it does not occupy any farming land or forest reserves.

Industrial production of algae suitable for producing renewable oil requires large scale growth processes using selected high lipid containing microalgae strain, recovery of produced biomass from dilute solutions, separation of the desired product from the biomass and various purification steps.

Harvesting microalgae biomass is challenging. Major problems are the small size of the microalgae, tendency to grow as single cells and the low cell density in the culture medium. The solution volumes to be treated are large and the amount of processing and energy needed renders the separation of pure microalgae biomass demanding and expensive. It has been calculated that up to 20-30% of the total production costs are due to biomass recovery stage.

The amount of water to be drained is large and the separation of desired algae, for example, using centrifugation or drying is expensive. Centrifugation is widely used and effective, but leads to extremely high capital and operational costs.

Methods suitable for macroalgae are generally not applicable for collecting microalgae i.e. microscopic algae of cell size below about 20 µm or cultured microalgae of cell size even less than 10 µm. In addition to the small size and active swimming behavior using flagella, the existence of cell wall, its thickness and material in many unicellular microalgae influence their sedimentation. For example, larger (>10 µm) diatoms with thick siliceous cell wall have higher sedimentation rate compared to very small (2-5 µm) flagellated cells with thin or non-existent cell wall.

After the growth of microalgae in dilute growth solutions the biomass is separated aiming at 50-200 times concentrated biomass. Eventually, biomass should have dry weight of about 5-15% by weight.

Methods used in water purification such as flocculation or coagulation may be applied to harvesting biomass, as well. One drawback in the used flocculation methods is that the yield of recovered biomass remains low, typically only about 80%. Moreover, the separating processes are slow in removing water and recovering the usable biomass with low water content.

US 2009/0162919 discloses a commercially viable and large scale method for concentrating microalgae having a cell diameter less than 20 µm in an aqueous environment. In this method microalgae are contacted with an inorganic flocculant, preferably aluminum flocculant such as polyaluminum chloride, forming flocs which are subsequently separated. This flocculated microalgae form concentrated slurry with a biomass density of at least 1%. In some of the embodiments additionally organic polymer such as monomer of acrylamide, acrylate, amine or a mixture thereof is further added into the microalgae solution. This organic polymer may be derived from a naturally occurring material, for example, chitosan or clay. Harvesting efficiencies above 80% are achieved. The amount of iron and aluminium flocculants used in the examples may hinder further use of algae biomass thus obtained for applications requiring low or no metal content.

The object of the present invention is to provide a method suitable for efficiently harvesting algae, especially microalgae, from dilute aqueous growth solutions.

A further object of the present invention is to provide a robust method for efficiently harvesting algae economically at large scale with high yield using as low amounts of chemicals as possible.

### Summary of the invention

The inventors have found that surprisingly good microalgae yields with a very short process duration are obtained by introducing an organic coagulant and inert inorganic clay stepwise in a specific order into an aqueous solution containing the finely suspended microalgae.

In one aspect, the present invention provides a method for harvesting algae as depicted by claim 1.

The use of iron and/or aluminium based flocculants may hinder further use of the algae biomass, for example, in food or feed applications. The metal content should remain below allowable or recommended limits. Moreover, the use of excess metals should be avoided since they may cause problems, for example, if the algae mass is to be used as a source for fuel production or as fish fodder. Metals in oils extracted from biomass typically need to be removed before further processing. Residue metals in the water phase also limit the recycling of the growth medium and may require additional purification steps. The current invention therefore provides a method for harvesting algae from an aqueous solution, which requires less purification of algae mass before further use.

The effect of the used stepwise method of adding organic coagulant and inert inorganic clay may be further enhanced by adding a small amount of an inorganic flocculant into the algae containing solution after the treatment with organic coagulant and inert inorganic clay. The addition of inorganic flocculant facilitates to reduce the amount of organic coagulant and inert clay needed depending on the algae strain. Moreover, the total time for flocculation can be reduced and larger flocs are formed when additional inorganic flocculant was used. However, for certain applications the use of iron and/or aluminium containing flocculants is not feasible. For certain application a low amount of metal containing flocculants is possible but preferably to be avoided, or at least the amount must be low enough not to cause problems with further processing.

Mixing in connection with the chemical addition in steps (i) and (ii) is necessary for providing efficient contact between the chemicals and algae specimen and to boost the floc formation effect. The addition steps of the chemicals are followed by mixing and subsequently an agitation step. Flocs will mainly be formed during the agitation.

The produced flocculated algae are separated and collected giving a surprisingly good yield. More than 90% of the algae originally in the aqueous solution to be treated are recovered, preferably more than 95%, more preferably even more than 99%. The volume of the separated and collected algae mass in the method according to the present invention is at least 10%, preferably at least 5%, most preferably at least 1%, such as 0,5%, of the volume of the original algae containing aqueous solution. Furthermore, the method provides a very fast overall processing time. As an example, a one liter batch could be processed in less than 10 minutes. The process and equipment used are readily scalable up into continuous operation mode of desired quantities of tens of tons per hour.

### Brief description of the drawings

Figure 1 shows a preferred embodiment of a harvesting scheme according to the present invention.
Figure 2 shows another preferred embodiment of a harvesting scheme according to the present invention.

### Detailed description of the invention.

Industrial scale algae growth may be realized in several ways. Culturing in fresh water ponds free from salts facilitates a harvesting process whereas using sea water ponds, and especially higher salinity ponds, is challenging due to possible precipitation of salts, such as Mg and/or Ca salts, from the growth solution when the solution properties are altered. Salinity also increases the density of the growth medium, and the relative density difference between microalgal cell and water becomes very small complicating the use of centrifugation or settling of cells. Furthermore, coprecipitation together with algae is likely to occur when pH needs to be adjusted for precipitation. Open pond may comprise an area of several square kilometers. Algae culture ponds are likely to suffer from impact due to external factors such as dependence of growth rate of each separate micro-organism on illumination and temperature of the ambient, carbon dioxide dissolution from surrounding air altering the solution pH, contamination by e.g. sand, other bacteria and microorganisms, dead cells and/or other unexpected unavoidable open air phenomena. The present invention offers a robust method suitable for use even for high salinity open pond originating algae solutions.

The method of the present invention is suitable for autotrophically, heterotrophically or mixotrophically grown algae, preferably for microalgae which are single cell algae and invisible to naked eye i.e. less that 50 µm. Preferably, suitable microalgae comprise one or more representatives from the following taxonomic classes: Chlorophyceae (recoiling algae), Dinophyceae (dinoflagellates), Prymnesiophyceae (haptophyte algae), Pavlovophyceae, Chrysophyceae (golden-brown algae), Diatomophyceae (diatoms), Eustigmatophyceae, Rhapidophyceae, Euglenophyceae, Pedinophyceae, Prasinophyceae and Chlorophyceae. More preferably, microalgae genera comprise *Dunaliella, Chlorella, Botryococcus, Haematococcus, Chlamydomas, Isochrysis, Pleurochrysis, Pavlova, Phaeodactylum, Skeletonema, Chaetoceros, Nitzschia, Nannochloropsis, Tetraselmis and Synechocystis.* Most preferably, microalgae are selected from the group consisting of *Dunaliella, Chlorella, Botryococcus, Haematococcus, Nannochloris, Chlamydomas, Isochrysis, Pleurochrysis, Pavlova, Phaeodactylum, Skeletonema, Chaetoceros, Nitzschia, Nannochloropsis, Tetraselmis and Synechocystis.* The method was found to be particularly effective with microalgae selected from the group consisting of *Nannochloropsis sp.* such as green algae *Nannochloropsis sp.* grown in sea water, which is a spherical picoalgae having a shell comprising polysaccharides and producing polyunsaturated fatty acids; *Haematococcus sp.; Dunaliella sp.* such as green algae *Dunaliella tertiolecta; Phaeodactylum sp.* such as green algae *Phaeodactylum tricornutum;* and *Chlorella sp.* such as green algae *Chlorella ypenoidosa* capable of incorporating a high lipid content but difficult to recover by mere use of flocculating agents.

The microalgae culture may be a pure population but is typically a mixed population, especially in an open pond. Suitable microalgae are naturally occurring micro-organisms, bred or engineered micro-organisms. The present robust method allows the use of various types of mixed microalgae populations even with a degree of contamination therein. An advantage of the present method is that microalgae which do not easily form aggregates such as filaments or colonies, and which do not spontaneously settle from the culture medium, especially even those microalgae that are particularly small and have flagellated cells with thin or non-existent cell wall, can be flocculated and effectively harvested from the aqueous solution.

The chemical composition and pH of the algae growth solution are dependent on the algae population and therefore react very differently towards the changes in temperature, pH, acid/base additions and added chemicals. This makes it difficult to predict the sedimentation behaviour of the algae. Preferably used algae are typically those grown in sea water type solutions having a salinity of sea water, up to 4,5 %. Best results are obtained with algae grown in salinity below 4% also depending on the algae strain. When the salinity is increased, precipitation of alkaline earth salts increases and hence the amount of chemicals needed increases. On the other hand, the resulting flocs are very resistant to mechanical handling. If the salinity of the growth solution is more than 4,5%, such as from 6 to 12% by weight, the solution to be treated can be diluted by fresh water or by sea water having salinity typically of less than about 3,5%, such as brackish water from estuaries.

The growth solution may have an alkaline pH as high as 7-9 whereto neutralizing agents typically need to be added in order to avoid unwanted precipitation of certain chemicals such as flocculation chemicals. The need for pH adjustment chemicals increases the processing costs. An attractive feature of the present robust method is that no pH adjustment of the growth solution is necessarily required. An algae containing aqueous solution used as the starting point in the method of the present invention may be any kind of aqueous solution suitable for maintaining algae culture i.e. cell structure and viability of the algae remains intact. The method is applicable with artificial culture media, as well as natural freshwater or seawater medium, with pH ranging from slightly acidic to basic, and a wide salinity range, preferably less than 4,5% by weight, more preferably less than 4%, most preferably less than 3,7%, such as less than 3,5%.

According to a preferred embodiment the aqueous algae growth solution or suspension is used as such in the method of the present invention.

The term "solution" refers to the liquid phase wherein the microalgae reside in the algae containing aqueous solution. Prior to the treatment the microalgae are more or less suspended in the solution forming a suspension which comprises the liquid phase and the microalgae. After the use of chemicals part of the algae form aggregates, agglomerates, microflocs or flocs resulting in a slurry formation wherein there may still be some suspended algae in the liquid phase as a suspension. All these various aqueous type of compositions are referred to as "solution".

The algae content in the growth solution is typically quite low to enhance the growth and photosynthesis. Pond depths up to 30 cm ensure favourable daylight proportion throughout the algae mass. In the method of the present invention the concentration of algae in said algae containing solution can be from 5 to 10% by weight for heterotrophically grown culture solutions in bioreactors. In an open pond cultivation the concentration is typically lower. The method of the present invention is successfully able to floc, separate and recover algae from algae containing aqueous solutions having the algae concentration of 1.5 g dry weight/I (dw/l), or even 1.0 g dw/I or yet even 0.8 g dw/l. For certain circumstances, it is even possible to use concentrations as low as 0.5 g dw/l. Preferably, the algae containing aqueous solution has a neutral pH or is slightly acidic or alkaline depending on the algae strain and growth phase in question. High photosynthesis rate during daytime for example is known to increase pH, while respiration decreases pH.

The first step of the method of the present invention involves addition of an organic coagulant into the algae containing solution. An advantage in using an organic coagulant is that it is not sensitive to the pH of the algae containing solution.

Preferably, the used organic coagulant is a highly cationic polymer coagulant. More preferably, the organic coagulant is selected from the group of polymers of dialkylaminoalkyl (meth)acrylates; polymers of dialkylaminoalkyl (meth)acrylamides; polymers of diallyldialkyl ammonium halides; polymers formed from an amine and epihalohydrin or dihalohydrin; and polyamides. Most preferably, the organic coagulant comprises epichlorohydrin dimethylamine copolymer. It is also possible to use a mixture of organic coagulants.

The amount of organic coagulant needed is typically less than 20 mg/l. Even less than 15 mg/l gives good results or even less than 10 mg/l. In one embodiment an amount less than 5 mg/l has been found sufficient, in particular, depending on the algae culture. In another embodiment an amount less than 3 mg/l is possible depending on the amount of clay and optionally added inorganic flocculants and/or polyelectrolytes. The amount of organic coagulant used should be as low as possible for cost reasons. The amount of organic coagulant is to some extent dependent on the algae type, the possible presence of mixed population, impurities, contamination and the properties of algae growth solution i.e. pH and salinity. Furthermore, the amount of organic coagulant is dependent on the amount of inorganic clay and/or possible inorganic flocculant to be added in the subsequent method steps. Preferably, the ratio of inert clay material in mg/l to organic coagulant in mg/l is from 100 to 5, preferably from 60 to 5, and most preferably from 45 to 15. The organic coagulant is typically a commercially known coagulant solution, which may be used as such, or is preferably first diluted by water prior to addition into the algae containing solution.

Organic coagulants are typically used in water purification systems at an acidic pH, such as about 4-5. It was surprisingly found that these chemicals could be used for algae aggregation in a wide pH range, even at high pH values such as 7-9. This is most convenient as no additions of further neutralising chemicals are needed for pH adjustment. The resulting solutions may be circulated without further chemical additions, thus minimizing the chemical consumptions.

Mixing of the resulting solution containing the algae and the organic coagulant is required for efficiently coagulating the algae and producing the desired properties for the subsequent flocculation, separation and collection of the algae. The mixing is preferably a vigorous mixing producing turbulent liquid flow compared to the later agitation phase which includes only a mild mixing.

In one embodiment, the mixing, preferably vigorous mixing, is continuous mixing, preferably carried out throughout the addition of the organic coagulant in step (i) and the addition of the inert inorganic clay material in step (ii), and continuing further after step (ii).

In another embodiment, mixing during and/or after addition of a chemical in step (i), and also in step (ii), is realized by creating a sufficiently strong mass flow for efficient mixing.

In yet another embodiment, mixing is achieved by means of static baffles, or the like, producing turbulent flow of solution inside e.g. the used pipe installation or tubing.

The total mixing time is dependent on the solution characteristics and volume and the apparatus used. As an example, a volume of 800 ml in a beaker is mixed for less than 5 min, preferably less than 1 min, more preferably from 10 to 60 sec, using vigorous mixing.

By "vigorous mixing" is meant mixing producing the same effect as mixing of 800 ml algae growth solution in 1 I beaker using a propeller with baffles and about from 200 to 400 rpm mixing rate. A commercial equipment for testing purposes is available by, for example, the company Kemira Kemwater called the MiniFlocculator. A person skilled in the field of mixing liquids is able to scale up the mixing.

According to a preferred embodiment the duration of the mixing, preferably vigorous mixing, after addition of inert organic coagulant is equal to or longer than the duration of the agitation phase in step (iii).

Without being bound by any theory, efficient mixing in connection with the organic coagulant is considered necessary for providing efficient contact between the coagulant and algae specimen and to facilitate a fast formation of algae aggregates or small agglomerates. No visible flocs are necessarily formed. The size of agglomerates or aggregates is still substantially below the visible detection limit.

In the subsequent or second step of the method inert inorganic clay material is provided to the solution obtained from the first step. This inert inorganic clay is preferably inert inorganic clay material originating from industrial processes as waste, such as gypsum from sulphur removal processes. More preferably the inert clay material comprises bentonite, kaolin, diatomite or modified diatomite, limestone and/or gypsum, most preferably bentonite such as natural or acid activated bentonite. The clay material may additionally comprise quartz, calcite, attapulgite, paly-gorskite, muscovite, dolomite, halloysite or silica.

The amount of clay material needed is less than 1000 mg/l. Typically, already smaller amount of less than 500 mg/l produces good results. An amount of less than 125 mg/l has been found sufficient or even less than 90 mg/l. Depending on the circumstances and the algae culture, amount of less than 50 mg/l has been found effective. The aim is to minimize the amount of clay to be used, such minimization being within the expertise of a person skilled in the field of flocculation and being able to vary the amount depending on the amounts of organic coagulant and possible other chemicals used. The inert inorganic clay material is preferably first mixed with water and subsequently fed into the algae containing solution as a slurry.

The addition of the clay material initiates the formation of visible algae containing flocs. However, the most of the flocs are formed during the following agitation step.

Mixing is still required after providing the inert clay material and organic coagulant into the aqueous solution of algae for efficiently flocculating the algae.

The chemical additions and mixing phases of step (i) and step (ii) are followed by a gentle agitation phase before the collection of the flocculated algae. Preferably, the gentle agitation phase of step (iii) is performed at an agitation mixing rate which is about 1/10 of the mixing rate of steps (i) or (ii), preferably the vigorous mixing rate of steps (i) or (ii). The typical duration of the agitation phase is less than 10 minutes, preferably less than 5 minutes, more preferably from 0,5 to 2 minutes, most preferably about one minute. The delay time and mixing rate in vigorous mixing and gentle agitation may be used to adjust the microalgae agglomeration depending on the culture quality. Sometimes the floc formation occurs immediately after the addition of the flocculant. Feedback in a continuous process is obtainable by only a short delay as the method steps can be carried out in a short duration of time.

Optionally, after step (i), preferably during the agitation phase (iii), an inorganic flocculant is added to the algae solution. Typical commercial flocculants may be used. Traditionally, inorganic flocculants, such as alum, ferric chloride, ferrous sulphate and lime, have been used. Preferably, the inorganic flocculant comprises an iron compound and/or an aluminium compound. More preferably, the iron compound is ferric salt such as ferric chloride, ferric sulfate, or ferrous sulphate, most preferable ferric sulphate. The aluminium compound is more preferably an aluminium salt, such as aluminum chloride, aluminum sulfate, polyaluminum chloride, aluminum chlorohydrate, or sodium aluminate, most preferably polyaluminium chloride. Oxidising chemicals such as hydrogen peroxide, may be provided together with iron compound to enhance the flocculation effect.

When using first the organic coagulant and subsequently the inorganic clay, the amount of inorganic flocculant needed is low compared to the traditional flocculation provided only by the use of inorganic flocculant as the primary or only flocculating agent. Thus, in the present inventive method the amount of metal compounds to be added into the algae containing solution can be minimized.

In a preferred embodiment the amount of inorganic flocculant used is less than 2 mg/l in the algae containing solution. Preferably, only a gentle agitation is applied after addition of the inorganic flocculants.

In one embodiment polyelectrolytes are added in addition to the iron compounds and/or the aluminium compounds. These chemicals typically enhance the floc size, even flocs with 1 cm may be obtained and the flocs are more easily separated from the solution. Moreover, their use is found especially advantageous in contaminated conditions or when mixed populations are concerned. Polyelectrolytes are available from commercial manufacturers. Preferably, polyacrylamide copolymers are used, more preferably copolymers of acrylamide and sodium acrylate or amine. Polyelectrolytes are preferably provided in an amount of less than 20 ppm, more preferably less than 10 ppm and most preferably 0.5 ― 2.5 ppm, as a very dilute solution such as for example 0.05% by weight solution. Polyelectrolytes are most preferably added during the agitation phase.

The total duration for collecting the algae using the present method is less than 60 min, preferably less than 30 min, more preferably less than 15 min, most preferably less than 10 min.

The final step of the method is separating the formed algae flocs from the solution phase and collecting them for further processing or use. Depending on the type of flocs generated typical means for separation and collecting the flocs are used. Preferably, the recovery is performed by sedimentation or flotation. Flotation is preferred when the surface area is large, as collection of algae is thus more efficient. Flotation may be assisted or natural depending on the properties of the algae flocs, such as the amount of lipids therein or their response to ambient light or other solute specimen conditions. Pressurized water is commonly used for flotation. Preferably, the flotated algae flocs are skimmed off from the solution surface. Further treatment by centrifugation, drum filtering or wire filtering is enabled by the diminished water content of the skimmed algae flocs.

The amount of solution or water in the produced algae floc mass is low, preferably less than 5% by weight, more preferably less than 4% by weight, most preferably less than 3% by weight, such as about 1%, which facilitates the efficient recovery of the desired components for further processing. Thus, the biomass may be concentrated 160 fold or even more compared to the original weight percent.

The remaining algae solution and/or supernatant may be recirculated back.

The algae solution and/or supernatant remaining after separation of algae floc are typically measured optically to determine the harvesting yield. The algae cell amount, i.e. optical density (OD), is conveniently determined by measuring the green color intensity at 680 nm wavelength by spectrophotometer. The optical density measured from the supernatant after collecting the algae floc by the method of the present invention is less than 10% compared to the untreated solution i.e. 90% of the algae is collected. Preferably, up to 95% is collected, most preferably up to 99% depending on the algae and chemicals used.

Clearly, a synergistic effect is observed when first, adding the organic coagulant and secondly, adding the inert inorganic clay. Judging from the OD measurements on algae amount still remaining in the solution after removal of flocs, it is evident that mere addition of organic coagulant or inert inorganic clay alone does not result in reasonable OD values. The removal efficiency of algae is several tens of percentage units better when the sequence of the present invention is used.

A similar effect can be seen with the addition of inorganic flocculants. Considerably higher amounts are required in order to obtain agreeable OD values or those comparable to OD values obtained with combined additions of organic coagulant and inert inorganic clay. The additional advantage obtained with addition of inorganic flocculants or polyelectrolytes may be compromised to decrease the amount of organic coagulant and inert inorganic clay material to be used to reduce the overall amount of the chemicals.

This synergy effect is discussed further in the following examples.

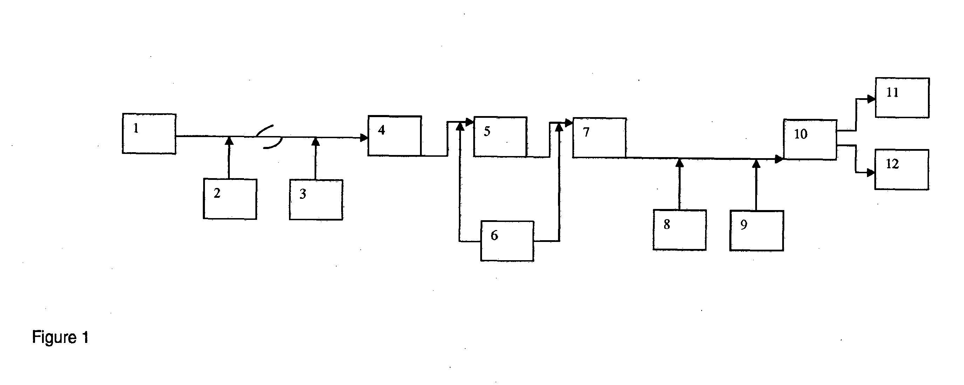

Figure 1 shows an example of a process flow according to the invention. Algae containing solution is continuously drawn from a pond 1 into a tubing wherein first organic coagulant 2 is added and mixed into the algae containing solution via a static mixer. Subsequently, a slurry of inert inorganic clay 3 is introduced and the solution is led into a reservoir 4 which is equipped with mixing means suitable for fast, vigorous mixing. The liquid level of the reservoir is used for adjusting the retention time. The formed algae floc solution is led into an intermediate reservoir 5 for additional mixing preceding an introduction of inorganic flocculant 6 and/or introducing inorganic flocculant 6 after additional mixing. The algae floc solution is led to a reservoir 7 which is equipped with mixing means suitable for gentle mixing or agitation. Subsequently, polyelectrolyte solution 8 is added with dispersion water 9 and the solution is transported into flotation unit 10 for the removal of algae flocs 11 and water 12, which may be recycled back to e.g. a pond or a culture solution.

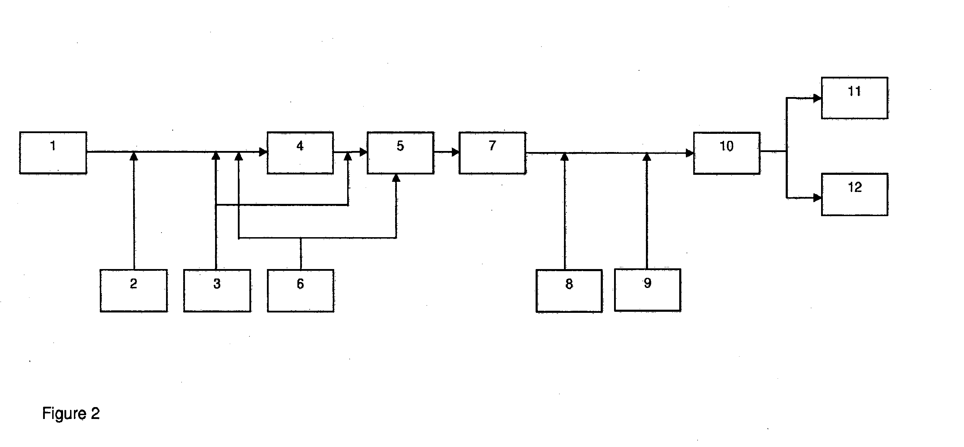

Figure 2 shows another example of a process flow according to the invention. Algae containing solution is continuously drawn from a pond 1 into a tubing wherein the first organic coagulant 2 is added and mixed into the algae containing solution through turbulent pump flow. Subsequently, a slurry of inert inorganic clay 3 is introduced and the solution is led into a reservoir 4 which is equipped with mixing means suitable for fast, vigorous mixing. The liquid level of the reservoir is used for adjusting the retention time. The inorganic flocculant 6 is introduced into the solution flow before it is led into the reservoir 4. Further slurry of inert inorganic clay 3 is introduced into the solution before it is led into an intermediate reservoir 5 for additional mixing. Further inorganic flocculant 6 is introduced into an intermediate reservoir 5. The algae floc solution is led to a reservoir 7 which is equipped with mixing means suitable for gentle mixing or agitation. Subsequently, polyelectrolyte solution 8 is added with dispersion water 9 and the solution is transported into flotation unit 10 for the removal of algae flocs 11 and water 12, which may be recycled back to e.g. a pond or a culture solution.

The following examples illustrate the inventive method at various conditions. These examples are illustrative only and not intended to be limiting in scope.

### Examples

### Example 1

Harvesting experiments were performed in a 1000 ml beaker with 800 ml microalgae culture. This was equipped with a blade stirrer with an adjustable stirrer speed region 200-400 rpm and region 10-50 rpm. The equipment is sold under name MiniFlocculator by Kemira, Kemwater.

This example demonstrates the successful concentration and separation of microalgae of the genus *Chlorella,* solution salinity 1,4% and pH 4,9 and biomass concentration 1,00 g/I, by first coagulating and flocculating the microalgae with an organic coagulant and inert clay material and then sedimenting the microalgae.

The organic coagulant, Fennofix 50 (from the company Kemira) was dissolved in water at a concentration of 5 g/I. The inert clay, bentonite (Berkbond No. 2, Steeley Minerals Division, Milton Keynes, UK), was slurried in water having about 5% dry matter.

The inorganic flocculant 1, Fennoferri 105 (from the company Kemira), was dissolved in water at a concentration of 4 g/I ferric sulphate. The inorganic flocculant 2, Kempac 18 (from the company Kemira) was dissolved in water at a concentration of 3,5 g/I polyaluminiumchloride.

First, the organic coagulant was added to the *Chlorella* microalgae culture having a biomass density of 1 g/I. The solution was agitated vigorously for up to 3 min. Secondly, the inert clay was added. The solution was agitated vigorously for 0,25-3 min and then more gently until flocs were formed. This required up to 5 min. The formed flocs sedimented, depending on the floc size, from 15 sec to 15 min.

In some of the experiments the inorganic flocculant 1, Fennoferri 105, was added after the addition of inert clay.

The performance of the sedimentation-based harvesting process was judged based on settling speed, removal efficiency (algae left in suspension) and density of the obtained concentrate (OD680%, measure optically at 680 nm). A summary of the results obtained with this procedure is shown in Table 1.

**Table 1**

| Experiment | Organic coagulant (mg/l) | Inert inorganic clay (mg/l) | Inorganic flocculant 1 (mg/l) | Ratio clay/coagulant | OD680-% |
|---|---|---|---|---|---|
| 1 | 6,25 | 125 | | 20 | 7,4 |
| 2 | 12,5 | 250 | | 20 | 1,6 |
| 3 | 5 | 500 | | 100 | 4,8 |
| 4 | 6 | 250 | 1,0 | 42 | 2,7 |
| 5 | | | 40,0 | | 8,6 |

The flocculation was effective with a small amount of coagulant and clay. Addition of clay made the flocculated algae dense and it occupied only 3% of the original volume. The addition of a very small amount of flocculant 1, as shown in experiment 4, decreased further the needed chemical amounts.

It is further noted that an excellent result is obtained when only using the combination of first adding the organic coagulant and subsequently adding the inert inorganic clay, as is shown in experiment 2, without any further addition of an inorganic flocculant.

In comparison, when flocculating the algae with only inorganic flocculant, as shown in experiment 5, the amount needed to flocculate and sediment 91,4% of the algae (8,6% left in suspension) was 40 mg/l and the flocculated algae occupied a volume of 5 % of the original.

### Example 2

Harvesting experiments were performed similarly to example 1 with the exception of using the microalgae of the genus *Dunaliella* with a solution salinity 3,5%, pH 8,5-9 and biomass concentration 0,2 g/I.

The performance of the sedimentation-based harvesting process was judged based on settling speed, removal efficiency (algae left in suspension) and density of the obtained concentrate. A summary of the results obtained with this procedure is shown in Table 2.

**Table 2**

| Experiment | Organic coagulant (mg/l) | Inert inorganic clay (mg/l) | Inorganic flocculants 1 (mg/l) | Ratio clay/coaqulant | OD680-% |
|---|---|---|---|---|---|
| 1 | 12,5 | 750 | | 60 | 3,7 |
| 2 | 12,5 | 500 | | 40 | 7,2 |
| 3 | 18,75 | 500 | 1,0 | 27 | 4,7 |
| 4 | 18,75 | 250 | 2,0 | 13 | 4,3 |
| 5 | | 500 | | | 41,9 |
| 6 | 25 | | | | 31,9 |
| 7 | | | 35 | | 5,1 |

Flocculation using only the organic coagulant was found ineffective, as shown in experiment 6, resulting in very small flocs with slow settling rate leaving 31,9% of the algae in suspension when 25 mg/l of organic coagulant was added.

An addition of only 500 mg/l clay, as shown in experiment 5 resulted in some algae removal but 41,9% of algae was left in suspension.

The combination of coagulant and clay, as shown in experiments 1 and 2, made the flocs heavy and settling fast (5 min). Addition of a very small amount of flocculant 1, as shown in experiments 3 and 4, made the flocs still more dense and removal more efficient.

In comparison, when the inorganic flocculant was added alone, as shown in experiment 7, a much larger amount of 35 mg/l was needed to remove 95% of the algae.

### Example 3

Harvesting experiments were performed similarly to example 1 with the exeption of using the microalgae of the genus *Nannochloropsis,* in a solution salinity 2,9%, pH 6,1 and biomass concentration 0,2 g/I.

The performance of the sedimentation-based harvesting process was judged based on settling speed, removal efficiency (algae left in suspension) and density of the obtained concentrate. A summary of the results obtained with this procedure is shown in Table 3.

**Table 3. Nannochloropsis**

| Experiment | Organic coagulant (mg/l) | Inert inorganic clay (mg/l) | Inorganic flocculant 1 (mg/l) | Inorganic flocculant 2 (mg/l) | Ratio clay/coagulant | OD680-% |
|---|---|---|---|---|---|---|
| 1 | 18,75 | 500 | | | 27 | 3,1 |
| 2 | 18,75 | 125 | 4 | | 7 | 4,3 |
| 3 | 12,5 | 500 | 0,5 | | 40 | 1,9 |
| 4 | 12,5 | 312,5 | 1,5 | | 25 | 3,3 |
| 5 | 9,4 | 250 | 1 | | 27 | 5,8 |
| 6 | 6,25 | 375 | | | 60 | 13,7 |
| 7 | 6,25 | 375 | 1,5 | | 60 | 4,1 |
| 8 | 6,25 | 375 | 4 | | 60 | 7,4 |
| 9 | 6,25 | 375 | | 1,3 | 60 | 7,8 |
| 10 | 18,75 | | | | | 37,3 |
| 11 | | 500 | | | | 92 |
| 12 | 12,5 | | 1,25 | | | 27,9 |
| 13 | | 375 | 5,0 | | | 74,1 |
| 14 | | | 25 | | | 18,0 |
| 15 | | | 50 | | | 4,3 |
| 16 | | | | 22 | | 7,8 |
| 17 | | | | 44 | | 3,9 |

The coagulant (experiment 10) or the clay (experiment 11) alone or in combination with a small amount of inorganic flocculant (experiments 12 and 13) was not effective for removal of algae from the suspension. Whereas, the combination of first adding the coagulant and subsequently adding the clay worked well, as shown by the experiments 1 and 6. A small amount of the inorganic flocculant improved the result further, as shown in experiments 2-5 and 7-9.

In comparison, the amount of inorganic flocculant needed to flocculate more than 95% of the algae was 50 mg/l of flocculant 1 (experiment 15) or 44 mg/l of flocculant 2 (experiment 17). In comparison, flocculant 2 with a higher charge density was needed less than flocculant 1.

### Example 4

Harvesting experiments were performed similarly to example 1 with the exeption of using the microalgae of the genus *Phaeodactylum* in a solution salinity 3,5%, pH 8,5-9 and biomass concentration 0,4 g/l.

The performance of the sedimentation-based harvesting process was judged based on settling speed, removal efficiency (algae left in suspension) and density of the obtained concentrate. A summary of the results obtained with this procedure is shown in Table 4.

**Table 4. Phaeodactylum.**

| Experiment | Organic coagulant (mg/l) | Inert inorganic clay (mg/l) | Inorganic flocculant 1 (mg/l) | Ratio clay/coagulant | OD680-% |
|---|---|---|---|---|---|
| 1 | 9,4 | 62,5 | | 7 | 1,9 |
| 2 | 3,75 | 62,5 | | 17 | 5,5 |
| 3 | 6,25 | 125 | | 20 | 0,3 |
| 4 | 6,25 | 94 | | 15 | 1,0 |
| 5 | 3,125 | 125 | 1,25 | 40 | 0,5 |
| 6 | 3,125 | 94 | 3,75 | 30 | 0,5 |
| 7 | | | 25 | | 2,8 |

The combination of coagulant and clay worked very well also with algae of the genus *Phaeodactylum.*

### Example 5

Harvesting experiments were performed similarly to example 1 with the exeption of using the microalgae of the genus *Nannochloropsis* in a solution salinity 3,5%, pH 7,8 and biomass concentration 0,35 g/I

The performance of the sedimentation-based harvesting process was judged based on settling speed, removal efficiency (algae left in suspension) and density of the obtained concentrate. A summary of the results obtained with this procedure is shown in Table 5.

**Table 5. Nannochloropsis.**

| Organic coagulant (mg/l) | Inorganic inert clay (mg/l) | Flocculant 1 (mg/l) | OD680-% after | OD680-% after | Observations |
|---|---|---|---|---|---|
| | | | 30 min | 16h | |
| 12,5 | 62,5 | 2 | 2,9 | | Flocs form, settled in 5 min. |
| 12,5 | 62,5 | | 6,5 | | Small flocs form, settled in 15 min. |
| 12,5 | | 2 | no settling | 9,0 | Small flocs, very slow settling (more than 1 h, OD680 measured after 16 h) |
| 12,5 | | | no settling | 12,0 | Just visible small flocs, no settling in 1 h (OD680 measured after 16 h) |

Already a small clay addition made the flocs very much larger and settle faster. A small inorganic flocculant addition increased the floc size (1-3 mm) and enhanced settling from 15 min to 5 min. The organic coagulant on its own and together with a small inorganic flocculant addition formed very small flocs (just visible by eye) that did not settle in 30 min.

The organic coagulant did not function as a flocculant on its own. A small addition of clay made the flocs very much larger and settle fast. The inorganic flocculant addition made the settling even faster.

### Example 6

Increased floc size and strength was gained when organic polyelectrolyte was added during the gentle agitation after introduction of the organic coagulant and inorganic inert clay. Harvesting experiments were performed similarly to example 1 with the exeption of using the microalgae of the genus *Nannochloropsis,* salinity 2,9%, pH 6,1 and biomass concentration 0,2 g/l. The organic polyelectrolyte (Fennopol A321 from Kemira) suspended in water at a concentration of 0,05% was added during the gentle agitation.

After sedimentation, 30 min of settling, the culture solution was vigorously remixed (200 rpm, 30 s) and let to sediment again in order to measure the strength of the formed flocs.

**Table 6. Nannochloropsis.**

| Organic coagulant (mg/l) | Inorganic inert clay (mg/l) | Inorganic flocculant 1 (mg/l) | Inorganic flocculant 2 (mg/l) | Organic polyelectolyte (mg/l) | OD680-% after flocculation and settling 30 min | OD680-% after remixing (200 rpm 30 s) and settling 30 min |
|---|---|---|---|---|---|---|
| 6,25 | 375 | | 1,3 | | 7,76 | 9,01 |
| 6,25 | 375 | | 1,3 | 2,5 | 5,13 | 5,63 |
| 6,25 | 375 | 1,5 | | | 11,76 | 13,89 |
| 6,25 | 375 | 1,5 | | 2,5 | 4,63 | 4,51 |

Large flocs (upto 10 mm) formed after addition of the organic polyeletrolyte. They settled much faster (less than 30 s) than the small ones (ca. 1 mm) which formed without the addition (needed ca. 15 min). During the remixing the flocs without organic polyelectrolyte broke into smaller flocs and the settling was slower than after flocculation. The flocs with organic polyelectrolyte settled very fast also after remixing. This indicates that flocs have high mechanical strength.

In other words, the addition of organic polyelectrolyte increased the settling and mechanical strength of the flocs, i.e. they did not break in the remixing experiment.

### Example 7

Harvesting experiments were performed similarly to example 1 with the exeption of using the microalgae of the genus *Nannochloropsis* in a solution salinity 2,9%, pH 6,1 and biomass concentration 0,2 g/l.

In all tests 12,5 mg/l of organic coagulant was added followed by the addition of 500 mg/l of varying inert clay material and 0,5 mg/l flocculant 1.

The settled culture was then remixed (200 rpm, 30 s) and settled again (30 min) in order to test the floc strength.

Table 7 shows the results of the experiments wherein the following clay materiarls were used:
Reference experiment 1: Reference experiment with original *Nannochloropsis*
Reference experiment 2: Reference experiment with no clay addition
Experiment 3: kaoline (from May & Baker Ltd)
Experiment 4: diatomite (from Merck)
Experiment 5: a mixture of acidified bentonite with SiO₂xA1₂O₃xnH₂O (Galleon Earth V2 Super from Ashapura Volclay Limited)
Experiment 6: a mixture of bentonite, CaSO₄ and quartz (from BASF)
Experiment 7: natural Ca-bentonite, acid activated (Tonsil 9192FF from Süd Chemie)
Experiment 8: bentonite (Berkbond No. 2, Steeley Minerals Division, Milton Keynes, UK)
Experiment 9: a mixture of muscovite, quartz, kaolinite and halloysite (diatomite 55-75%, aluminiumoxide 10-20 %, iron oxide 2-10%, from TAIKO)

**Table 7.**

| | after flocculation | after remix |
|---|---|---|
| | OD680-% | OD680-% |
| Reference experiment 1 | 100,0 | N.A. |
| Reference experiment 2 | 27,9 | 33,5 |
| Experiment 3 | 9,9 | 8,0 |
| Experiment 4 | 20,7 | 18,8 |
| Experiment 5 | 14,4 | 16,1 |
| Experiment 6 | 12,3 | 11,6 |
| Experiment 7 | 9,1 | 12,0 |
| Experiment 8 | 1,9 | 1,5 |
| Experiment 9 | 5,8 | 11,3 |

## Claims

1. A method for collecting algae from an algae containing aqueous solution comprising the steps of
(i) first, providing an organic coagulant to said solution and mixing said solution, and
(ii) subsequently, providing inert inorganic clay material to said solution after step (i) and mixing said solution to form coagulated algae, and
(iii) agitating the resulting solution after step (ii) to form flocculated algae, and
(iv) subsequently, separating and collecting the flocculated algae from said solution.

2. The method according to claim 1, wherein the duration of the mixing phase of steps (i) and (ii) is equal to or longer than the duration of the agitation phase in step (iii).

3. The method according to any one of the claim 1 or 2 wherein the duration of the mixing in steps (i) and (ii) is less than 30 min, preferably less than 20 min, more preferably less than 10 min, most preferably less than 7 min, such as less than 5 min.

4. The method according to any one of the claims 1-3, wherein the ratio (mg/l per mg/l) of inert clay material to organic coagulant is from 100:1 to 5:1, preferably from 60:1 to 5:1 and most ,preferably from 45:1 to 15:1.

5. The method according to any one of the claims 1-4, wherein the amount of organic coagulant is at least 2 mg/l and inert clay material at least 50 mg/l.

6. The method according to any one of the claims 1-5 wherein said microalgae classes comprise Chlorophyceae (recoiling algae), Dinophyceae (dinoflagellates), Prymnesiophyceae (haptophyte algae), Chrysophyceae (golden-brown algae), Diatomophyceae (diatoms), Eustigmatophyceae, Rhapidophyceae, Eugleno-phyceae, Pedinophyceae, Prasinophyceae and Chlorophyceae.

7. The method according to any one of the claims 1-6 wherein said microalgae genera are selected from the group consisting of *Dunaliella, Chlorella_{;} Tetra-selmis, Botryococcus, Haematococcus, Phaeodactylum, Skeletonema, Chaetoceros, Isochrysis, Nannochloropsis, Nannochloris, Pa vlova, Nitzschia, Pleurochrysis, Chlamydomas and Synechocystis,* more preferably selected from the group consisting of *Nannochloropsis, Haematococcus, Dunaliella, Phaeodactylum* and *Chlorella.*

8. The method according to any one of the claims 1-7 wherein said inert clay material comprises bentonite, kaolin, diatomite, limestone or gypsum, preferably bentonite.

9. The method according to any one of the claims 1-8 wherein said organic coagulant comprises a polymer coagulant, preferably a highly cationic polymer coagulant.

10. The method according to claim 9 wherein said organic coagulant is selected from the group of polymers of dialkylaminoalkyl (meth)acrylates; polymers of dialkylaminoalkyl, (meth)acrylamides; polymers of diallyldialkyl ammonium halides; polymers formed from an amine and epihalohydrin or dihalohydrin; and polyamides.

11. The method according to claim 9 wherein said organic coagulant comprises epichlorohydrin dimethylamine copolymer.

12. The method according to any one of the claims 1-11, wherein additionally an inorganic flocculant is added to said solution after step (i), preferably the inorganic flocculant is added during the agitation phase in step (iii).

13. The method according to claim 12, wherein said inorganic flocculant comprises a ferric compound and/or an aluminium compound, preferably ferric sulphate and/or polyaluminium chloride.

14. The method according to any one of the claims 1-13 wherein said separating in step (iv) is performed by sedimentation or flotation.

## Patentansprüche

1. Verfahren zum Einsammeln von Algen von einer algenhaltigen wässrigen Lösung, welches Verfahren die folgenden Schritte umfasst
(i) erstens, Bereitstellen eines organischen Koagulationsmittels für die Lösung und Mischen der Lösung, und
(ii) nachfolgend, Bereitstellen von inertem unorganischem Tonmaterial für die Lösung nach Schritt (i) und Mischen der Lösung zur Bildung von koagulierten Algen, und
(iii) Umrühren der resultierenden Lösung nach Schritt (ii) zur Bildung von ausgeflockten Algen, und
(iv) nachfolgend, Trennen und Einsammeln der ausgeflockten Algen von der Lösung.

2. Verfahren nach Anspruch 1, wobei die Dauer der Mischphase von Schritt (i) und (ii) gleich oder länger als die Dauer der Umrührphase in Schritt (iii) ist.

3. Verfahren nach einem der Ansprüche 1 oder 2, wobei die Dauer des Mischens in Schritt (i) und (ii) weniger als 30 Min., vorzugsweise weniger als 20 Min., bevorzugter weniger als 10 Min., am meisten bevorzugt weniger als 7 Min., wie beispielsweise weniger als 5 Min. ist.

4. Verfahren nach einem der Ansprüche 1 bis 3, wobei das Verhältnis (mg/L pro mg/L) zwischen inertem Tonmaterial und organischem Koagulationsmittel von 100:1 bis 5:1, vorzugsweise von 60:1 bis 5:1 und am meisten bevorzugt von 45:1 bis 15:1 ist.

5. Verfahren nach einem der Ansprüche 1 bis 4, wobei die Menge an organischem Koagulationsmittel mindestens 2 mg/L und an inertem Tonmaterial mindestens 50 mg/L ist.

6. Verfahren nach einem der Ansprüche 1 bis 5, wobei Klassen von Mikroalgen Chlorophyceae (rückläufige Algen), Dinophyceae (Dinoflagellaten), Prymnesiophyceae (Haptophytalgen), Chrysophyceae (goldenbraune Algen), Diatomophyceae (Diatomeen), Eustigmatophyceae, Rhapidophyceae, Euglenophyceae, Pedinophyceae, Prasinophyceae und Chlorophyceae umfassen.

7. Verfahren nach einem der Ansprüche 1 bis 6, wobei die Mikroalgengattungen aus der Gruppe bestehend aus *Dunaliella, Chlorella, Tetraselmis, Botryococcus, Haematococcus, Phaeodactylum, Skeletonema, Chaetoceros, Isochrysis, Nannochloropsis, Nannochloris, Pavlova, Nitzschia, Pleurochrysis, Chlamydomas und Synechocystis,* bevorzugter aus der Gruppe bestehend aus *Nannochloropsis, Haematococcus, Dunaliella, Phaeodactylum* und *Chlorella* ausgewählt werden.

8. Verfahren nach einem der Ansprüche 1 bis 7, wobei das inerte Tonmaterial Bentonit, Kaolin, Diatomit, Kalkstein oder Gips, vorzugsweise Bentonit umfasst.

9. Verfahren nach einem der Ansprüche 1 bis 8, wobei das organische Koagulationsmittel ein Polymerkoagulationsmittel, vorzugsweise ein hochkationisches Polymerkoagulationsmittel umfasst.

10. Verfahren nach Anspruch 9, wobei das organische Koagulationsmittel aus der Gruppe bestehend aus Polymeren von Dialkylaminoalkyl(meth)acrylaten; Polymeren von Dialkylaminoalkyl(meth)acrylamiden; Polymeren von Diallyldialkylammoniumhalogeniden; Polymeren, die von einem Amin und Epihalohydrin oder Dihalohydrin gebildet sind; und Polyamiden ausgewählt wird.

11. Verfahren nach Anspruch 9, wobei das organische Koagulationsmittel Epichlorhydrin-Dimethylamin-Copolymer umfasst.

12. Verfahren nach einem der Ansprüche 1 bis 11, wobei ein unorganisches Flockungsmittel zur Lösung nach Schritt (i) zugesetzt wird, vorzugsweise wird das unorganische Flockungsmittel während der Umrührphase in Schritt (iii) zugesetzt.

13. Verfahren nach Anspruch 12, wobei das unorganische Flockungsmittel eine Eisenverbindung und/oder eine Aluminiumverbindung, vorzugsweise Eisensulfat und/oder Polyaluminiumchlorid umfasst.

14. Verfahren nach einem der Ansprüche 1 bis 13, wobei das Trennen in Schritt (iv) durch Ausfällung oder Flotierung durchgeführt wird.

## Revendications

1. Procédé pour la collecte d'algues à partir d'une solution aqueuse contenant des algues, comprenant les étapes consistant à
(i) en premier lieu, fournir un coagulant organique à ladite solution et mélanger ladite solution, et
(ii) par la suite, fournir un matériau d'argile inorganique inerte à ladite solution après l'étape (i) et mélanger ladite solution pour former des algues coagulées, et
(iii) agiter la solution obtenue après l'étape (ii) pour former des algues floculées, et
(iv) ensuite, séparer et recueillir les algues floculées à partir de ladite solution.

2. Procédé selon la revendication 1, dans lequel la durée de la phase de mélange des étapes (i) et (ii) est égale à ou plus longue que la durée de la phase d'agitation dans l'étape (iii).

3. Procédé selon l'une quelconque des revendications 1 ou 2, dans lequel la durée du mélange dans les étapes (i) et (ii) est inférieure à 30 min, de préférence inférieure à 20 min, plus préférablement inférieure à 10 min, le plus préférablement inférieure à 7 min, p.ex. inférieure à 5 min.

4. Procédé selon l'une quelconque des revendications 1 à 3, dans lequel le rapport (mg/l par mg/l) du matériau d'argile inerte au coagulant organique est de 100:1 à 5:1, de préférence de 60:1 à 5:1 et plus préférablement de 45:1 à 15:1.

5. Procédé selon l'une quelconque des revendications 1 à 4, dans lequel la quantité du coagulant organique est d'au moins 2 mg/l d'argile et de matériau inerte d'au moins 50 mg/l.

6. Procédé selon l'une quelconque des revendications 1 à 5, dans lequel lesdites classes de micro-algues comprennent Chlorophyceae (algues reculantes), Dinophyceae (dinoflagellés), Prymnesiophyceae (haptophyta algues), Chrysophyceae (algues brunes dorées), Diatomophycées (diatomées), Eustigmatophyceae, Rhapidophyceae, Euglenophyceae, Pedinophyceae, Prasinophyceae et Chlorophyceae.

7. Procédé selon l'une quelconque des revendications 1 à 6, dans lequel lesdits genres de micro-algues sont choisis dans le groupe constitué de *Dunaliella, Chlorella, Tetraselmis, Botryococcus, Haematococcus, Phaeodactylum, Skeletonema, Chaetoceros, Isochrysis, Nannochloropsis, Nannochloris, Pavlova, Nitzschia, Pleurochrysis , Chlamydomas et Synechocystis,* plus préférentiellement choisis dans le groupe constitué par *Nannochloropsis, Haematococcus, Dunaliella, Phaeodactylum* et *Chlorella.*

8. Procédé selon l'une quelconque des revendications 1 à 7, dans lequel ledit matériau d'argile inerte comprend la bentonite, le kaolin, la diatomite, le calcaire ou le plâtre, de préférence la bentonite.

9. Procédé selon l'une quelconque des revendications 1 à 8, dans lequel ledit coagulant organique comprend un coagulant polymère, de préférence un coagulant polymère fortement cationique.

10. Procédé selon la revendication 9, dans lequel ledit agent coagulant organique est choisi dans le groupe des polymères de dialkylaminoalkyl (méth)acrylates; des polymères de dialkylaminoalkyl (méth)acrylamides; des polymères d'halogénures d'ammonium diallyldialkyl; des polymères formés à partir d'une amine et d'une épihalohydrine ou dihalohydrine; et des polyamides.

11. Procédé selon la revendication 9, dans lequel ledit coagulant organique comprend un copolymère d'épichlorhydrine de la diméthylamine.

12. Procédé selon l'une quelconque des revendications 1 à 11, dans lequel en outre un floculant inorganique est ajouté à ladite solution après l'étape (i), de préférence le floculant inorganique est ajouté au cours de la phase d'agitation dans l'étape (iii).

13. Procédé selon la revendication 12, dans lequel ledit floculant inorganique comprend un composé ferrique et/ou un composé d'aluminium, de préférence le sulfate ferrique et/ou le chlorure de polyaluminium

14. Procédé selon l'une quelconque des revendications 1 à 13, dans lequel ladite séparation dans l'étape (iv) est effectuée par sédimentation ou flottation.
